# EUROPEAN PATENT APPLICATION

(11) **EP 1 031 356 A2**
(43) Date of publication of application: **30.08.2000**
(21) Application number: 00103737.3
(22) Date of filing: 22.02.2000
(51) Int. Cl.: A61L 31/04, A61L 31/14, A61L 27/24, A61L 27/50

(54) **Manufacturing methods of hollow fiber of narrow diameter and hollow fiber bundles**

(30) Priority: 23.02.1999 JP 4519099
(71) Applicant: DJK Research Center, Higashi Katsushika-gun, Chiba-ken (JP)
(72) Inventor: Nakagawa, Tokuzo, Kamakura-shi, Kanagawa-ken (JP); Kikuchi, Michihiro, Yokohama-shi, Kanagawa-ken (JP)
(74) Representative: TER MEER STEINMEISTER & PARTNER GbR

(57) **Abstract**

Disclosed is a hollow article such as a sheet composed essentially of a membraneous material and optionally other medically acceptable materials such as gelatin and collagen. The membraneous material consists only of acellular compact and basement membrane layers of mammalian connective tissue, is composed essentially of collagen bundles and has front and back surfaces that are asymmetric with each other. The hollow article typically has a plurality of open-ended cylindrical hollow spaces therein with an inside diameter of 1 µm to 5 mm and is useful, among others, as artificial neurilemma for wounded peripheral nerves.

## Description

### Field of the Invention

The invention relates to (1) a hollow fiber using a membraneous material consisting only of acellular compact and basement membrane layers of mammalian membraneous connective tissues, (2) bundles made of plural numbers of the hollow fibers and (3) their manufacturing methods. Particularly, the invention concerns hollow fibers and hollow fiber bundles that may be used in neurosurgery to treat quadriplegia caused by ablation of peripheral nerves occurring in wounds, contusion and other injuries. That is to say, the invention concerns hollow fibers and bundles thereof that may be used in the surgical field for restoring peripheral nerves that are cut during surgery, traffic accidents, labor accidents, sports accidents and the like. In addition, the hollow fibers and the bundles thereof may be used medically to improve biocompatibility and blood compatibility of implanting medical devices or self-retaining medical devices including, yet not limited to, artificial neurilemma (or nerve sheath), portable dialyzer, artificial glomerulus, artificial ligament, artificial tendon, artificial ureter, stent and the like.

Furthermore, by fixing cells, embryonic stem cells and other bioactive substances inside or outside the hollow fibers, the hollow fibers of the present invention may be used as a medical device to regenerate artificial liver, artificial pancreas and other artificial organs and tissues. The hollow fibers of the present invention may also be used for industrial applications such as in assay kits of environmental endocrine (hormone) disruptors, and as a module material for bioreactor in which petroleum is decomposed by microorganism. The invention is explained in detail below using artificial (neurilemma) as a typical example.

### Prior Art

Regeneration and restoration of peripheral nerves has been tried in various ways, one of which is a technique of transplanting peripheral nerves collected from cadavers. The technique has many serious problems that have to be solved including immunological rejection and preservation methods of peripheral nerves collected from cadavers. These problems remain unsolved and the practical use of the technique is far from reality.

A method has been proposed to regenerate peripheral nerves using as artificial neurilemma, a telopeptide collagen obtained by extracting from mammalian tissues and purifying to eliminate telopeptide. However, this technique has the following problems, which in reality remain unsolved.
1) When the mammal is cattle or sheep, there is a danger that a pathogen prion remains active. A method to inactive and/or eliminate the pathogen has not been established.
2) Collagen without telopeptide does not exist in and is heterogenous to a living body, there are various problems originating from such heterogenous collagen that inhibit regeneration of nerves. These problems belong to medical science and remain unsolved.
3) Problems have not been solved concerning residual antigenicity which is a serious problem for nervous regeneration, even though it might be trivial in other fields of art.
4) Manufacturing methods remain unsolved to form neurilemma in which a large number of tubules having an inside diameter of typically 0.1-0.2 mm, are bound together.
5) The problem of lack of extracellular matrix that is necessary for proliferation of nerve cells remains unsolved.

As described above, various techniques that make regeneration of peripheral nerves possible have been proposed. However, each technique has its own serious problems. Presently sought for is the appearance of simple techniques by which a stable supply is made possible.

### Problems the Invention Intends to Solve

In view of the current state described above, the inventors intended to solve problems that conventional techniques could not and to provide inexpensive artificial neurilemma made of a bundle of hollow fibers having an inside diameter of from about 1 µm to about 5 mm.

### Summary of the Invention

After having earnestly studied measures to solve the above described problems, the inventors have found a membraneous material comprising substantially the compact layer of mammalian connective tissue alone or a combination thereof with a fibrous substance that does not adhere to the membraneous material is particularly suitable for this purpose.

According to the present invention, a hollow fiber has at least one, preferably a plurality of hollow spaces therein and is composed essentially of membraneous material that comprises an acellular matrix which is a component of mammalian connective tissues, and optionally gelatin or collagen. The gelatin or collagen may be physically or chemically modified to crosslink it. Hollow fiber bundles can be made by winding spirally one or several of the hollow fiber layer. The hollow fiber bundles are useful as an artificial neurilemma as well as in other applications.

A particularly preferred embodiment provides a process for producing a hollow sheet, which comprises: (A) loading a membraneous material on a support member; (B) winding once a core fiber or a bundle of core fibers over the membraneous material and around the support member, wherein the core fiber or the bundle has a diameter of from 1 µm to 5 mm; (C) folding back the membraneous material along the core fiber or the bundle; (D) winding once the core fiber or the bundle over top and bottom layers of the folded back membraneous material and around the support member; (E) lifting the top layer of the membraneous material, winding once the core fiber or the bundle over the bottom layer of the membraneous material and around the support member and placing back the top layer of the membraneous material onto the core fiber or the bundle; (F) repeating the steps (D) and (E) a predetermined number of times, thereby obtaining an assembly of the membraneous material and the core fiber or bundle around the support member; (G) applying an aqueous gelatin or collagen solution or a medically acceptable adhesive to the membraneous material and the core fiber or bundle; (H) hardening the gelatin, collagen or medically acceptable adhesive; (I) cutting the core fiber or bundle where only membraneous material is absent, whereby obtaining a composite of the membraneous material, the core fiber or bundle and the gelatin, collagen or medically acceptable adhesive, removed from the support member; and (J) pulling out the core fiber or bundle from the composite, thereby obtaining the desired hollow sheet having a plurality of open-ended cylindrical hollow spaces having an inner diameter of 1 µm to 5 mm formed by the folded membraneous material and the gelatin, collagen or medically acceptable adhesive.

### Brief Description of the Drawings

Fig. 1 is a schematic view showing a preferred embodiment of the present invention; and
Fig. 2 is a schematic view showing another preferred embodiment of the present invention.

### Description of Preferred Embodiments

Hereinafter, preferred embodiments of the invention are explained in detail.

Human or mammalian membraneous connective tissues that are referred to in this specification include dura matter, pericardium, pleura, diaphragm, peritoneum, fascia lata, mesenterium, skin, tympanic membrane and other biogenic membranes, and vascular wall, esophageal wall, tracheal wall, urethra, ureteral wall, cardiac wall and external walls of other organs. Human or mammalian membraneous connective tissues also include fetal membrane and its structural materials, amnion and chorion. Human amnion is desirable material to be used for artificial neurilemma. There is no use currently of human amnion, which is treated as medical waste after childbirth. Moreover, human amnion can be collected at every childbirth, making the raw material to be acquired inexpensively and in a large quantity.

The structural configuration of connective tissue is dealt with in the science of physiology in functions by dividing it into four layers: epithelium, basement membrane, compact and fibroblast layers. Light-microscopically, connective tissue is divided visually into three layers: epithelium, basement membrane and fibroblast layers. That is to say, basement membrane and compact layers, the layers classified as two separate layers in physiology, are considered a one layer and called as basement membrane layer, light-microscopically. Epithelial and fibroblast layers are of cellular nature, while basement membrane and compact layers are of acellular nature. The thickness of the basement membrane layer is ultra-thin and expressed in nanometer (nm). The thickness of the compact layer is expressed in micrometer (µm).

Human amnion is approximately 12,000 µm thick, in which the boundary layers are basement membrane layer (50 to 80 µm thick) and compact layer (8,000 to 10,000 µm thick), and external sides are epithelial layer at one side and fibroblast layer at the other side. The substantially (or essentially) compact layer, as called in the specification, is a composite body comprising the compact layer and the basement membrane layer as they are called light-microscopically and is essentially compact layer, as is called in physiology.

The membraneous material that is essentially compact layer used according to the invention can be obtained as described in a following example. Biogenic connective tissue membrane consists of a membraneous layer which is essentially compact layer and epithelial layer and fibroblast layer on both sides which are cellular layers. The cellular membrane layers are made essentially of protein, just like bacterial cellular membrane. After biogenic connective membrane is collected and blood is eliminated in isotonic sodium chloride solution (i.e., physiological saline), the membrane is left in 0.1% benzalkonium chloride solution for 24 hrs or longer, in order to produce (electrical) potential degeneration of the cellular layers.

Next, in the environment at a pH value near neutrality, using enzymes with high capacity of proteolysis such as protease, the protein layers are decomposed in treatment at pH of about 7. Naturally, unless the decompositions of protein is controlled under appropriate conditions, the configuration of compact layer that is made up of collagen is also destroyed. Control factors include appropriate temperature and time to conserve morphological conditions of compact layer. The next step is ultrasonic cleaning. Ultrasonic cleaning eliminates the decomposition products of epithelial and fibroblast layers that adhered to compact layer. Thus, the membraneous material that comprises essentially compact layer is obtained.

The membraneous material is composed essentially of types I, III, IV, V and XVI collagen bundles and has front and back surfaces that are asymmetrical with each other.

Core fibers used in the invention are natural or synthetic macromolecular fibers or threads of a diameter of at least about 1 µm and not more than about 5 mm, preferably from 100 µm to 2mm. These fibers or threads preferably have poor affinity to a surface of the compact layer for easy removal, after the compact layer is made into a hollow tube shape. For these reasons, hydrophobic synthetic fibers, such as fluororesin especially polytetrafluoroethylene (e.g., Teflon - trade-mark) fibers are desirable. In general, natural macromolecular fibers are generally hydrophilic and their use is not favored. However, natural fibers can be used when a surface of them is treated with a water-repellent agent to make it hydrophobic. Fibers having a diameter of less than 1 µm is undesirable, as an inside diameter of the desired hollow fiber to be shaped becomes too small. Conversely, fibers having a diameter of larger than 5 mm, is not desirable, either, as the inside diameter of the desired hollow fiber is too large.

When the membrane strength of the compact layer used according to the invention is not sufficient, it is desirable to reinforce it using a fibrous material. The fibrous material used for this purpose must be biodegradable and bioabsorbable. Although polyglycolic acid, polylactic acid and their copolymers are desirable, other materials can be used.

As described above, human amnion is desirable to be used as human connective tissues according to the invention. The present invention offers a new use of human amnion that has been handled as medical waste at every childbirth. As human amnion can be collected at every childbirth, an advantage is that the raw material can be acquired inexpensively and in a large quantity. In addition, human-originated compact layer has almost no danger of generating biogenic immune reaction.

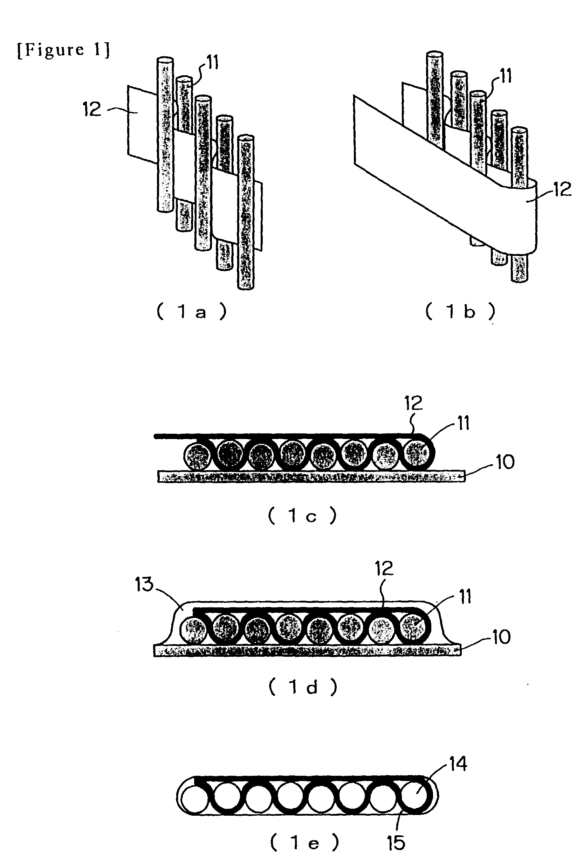

Hereinafter, using drawings, the manufacturing methods of hollow fiber bundles of the invention are described in detail. Fig. 1 shows an example of the manufacturing method according to the invention. A plurality of core fibers (11) are erected at equal distances, through which the membraneous material (12) is passed between each fiber in a zigzag fashion (Figure 1a). The remaining portion of the membraneous material is folded over one side of the row of the core fibers (Figure 1b). Then, tension is loaded on the core fibers from top and bottom. A body made up of the core fibers with the membraneous material is placed facing the folded membraneous material side up on a glass board (10) (Figure 1c). Water is sprayed to settle the form and the body is air-dried. After an aqueous gelatin solution or collagen solution is applied to the body until well incorporated, the body is heat-treated, for example, at 120°C for 24 hrs to harden gelatin or collagen (13) (Figure 1d).

After gelatin or collagen is hardened, the body is removed from the glass board and the core fibers are pulled out to obtain a hollow fiber, (15) with multiple parallel open-ended cylindrical hollow spaces, which looks like a bamboo blind (Figure 1e). Stating alternatively, it has a cross-section composed of a straight line and a waved (or corrugated) line connected together at bottoms of waves. The waved or corrugated portion has a radius that is about ½ of the diameter of the core fibers used for producing the hollow fiber, the product might not really be a "fiber", though it is described as a "hollow fiber" in this specification. It is in fact a relatively thin layer or sheet made of the membraneous material folded to form a plurality of open-ended cylindrical hollow spaces arranged in a row and having an inner diameter corresponding to the diameter of the core fibers. The thin layer may be reinforced by a biodegradable and bioabsorbable material, e.g., gelatin, collagen or the like. The thickness of the thin layer is slightly larger (say 10 - 100%) than that of the inner diameter of the hollow spaces. The number of the hollow spaces is preferably 2 to 50, more preferably 4 to 20. The length is not critical, but 1 to 30 cm is most practical.

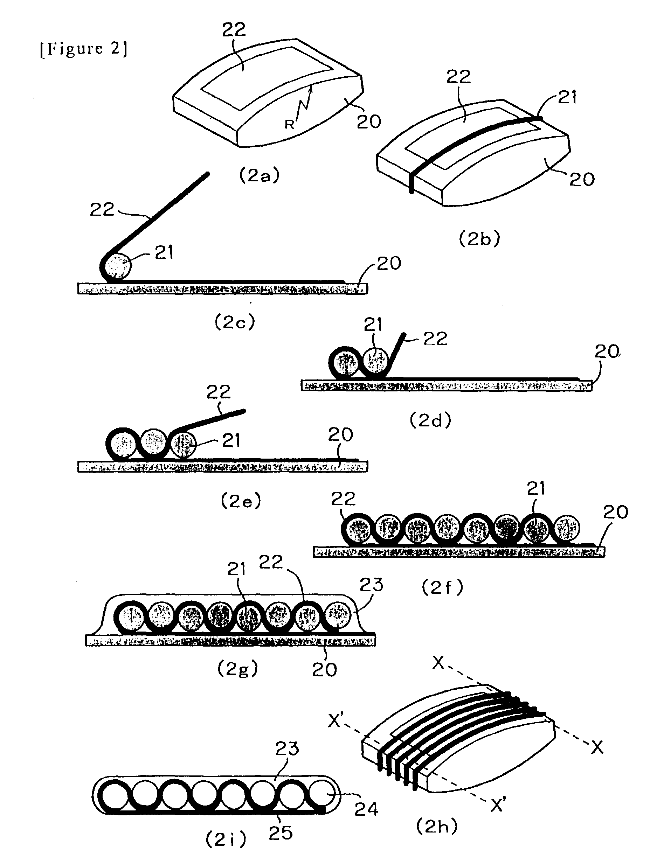

Figure 2 shows another example of the manufacturing method according to the invention. The membraneous material (22) is loaded on one side of a support member (20) having a spindle-shaped section (Figure 2a). A core fiber (21) wound around a separate bobbin (not shown) is wound over the membraneous material once around the support member (Figure 2b). The membraneous material is folded back along the core fiber (Figure 2c). The core fiber is wound once more over two layers of the membraneous material around the support member (Figure 2d). Next, while the membraneous material is lifted, the core fiber is wound once again over the bottom layer of the membraneous material around the support member and the top layer of the membraneous material is placed back onto the core fiber. The top layer of the membraneous material covers the core fiber (Figure 2e). By repeating the operation, the core fiber is wound a prescribed number of times (Figure 2f). Thus, an assembly of the core fiber and the membraneous material around the support member is obtained. After the whole assembly is air-dried, an aqueous gelatin solution or collagen solution is applied until well incorporated, followed by heat treatment, for example, at 120°C for 24 hrs to harden gelatin or collagen and to form a composite body (Figure 2g). In place of gelatin or collagen, a medically acceptable adhesive such as fibrin glue and cyanoacrylate may be used.

After gelatin or collagen is hardened, the core fiber is cut where the membraneous material is absent, e.g., the sites indicated by X and X', to remove the composite body from support member (Figure 2h). By pulling out the core fibers from the composite body, a bamboo blind-shaped hollow fiber (25) is obtained, in which hollow spaces of the same inside diameter (24) are lined up (Figure 2i).

Alternatively, a hollow fiber layer can be obtained by winding the membraneous material around single or multiple numbers of a core material, arranging many of the bodies in parallel, applying aqueous gelatin solution or collagen solution, hardening them and pulling out the core fibers.

By winding spirally the resulting hollow layers or sheets, hollow fiber bundles can be obtained.

Hereinafter, the invention is explained further in detail by working examples. Moreover, the present invention should not be considered to be restricted to these examples.

### Example I

### (1) Preparation of membraneous material

Human-originated amnion was washed repeatedly with isotonic sodium chloride solution (i.e., physiological saline) at room temperature. Manual washing was performed until it was confirmed macroscopically that blood was eliminated. Then, ultrasonic cleaning at 40 kHz was performed using running purified water (as prescribed in Japanese Pharmacopia) at room temperature for 12 hrs. After blood was eliminated, amnion was immersed in 0.1% benzalkonium chloride solution (as prescribed in Japanese Pharmacopia) and left stationary at room temperature for 24 hrs. Next, amnion was immersed in 0.2 M phosphate buffer solution containing 0.01% ficin and 0.05% sodium azide and left there stationary at room temperature for 24 hrs. By performing the above operation, epithelial and fibroblast layers of amnion were decomposed and eliminated. Obtained membraneous material was taken out and ultrasonic washing at 40 kHz was performed using running purified water at room temperature. Membraneous material comprising essentially acellular compact layer was obtained. The thus-obtained membraneous material was dried in a germ-free dryer at 35°C under reduced pressure for 12 hors. Thus, membraneous material, i.e., a raw material, was obtained.

### (2) Manufacture of hollow fiber layer

On a support member, 15 cm long and 10 cm wide, as shown in Figure 2, having a cross-section of a spindle shape, the membraneous material obtained in the above described method (1) 5 cm x 10 cm in size, was loaded. For a core fiber, Teflon (trade-mark) fiber of a diameter of 0.2 mm was used. The core fiber was wound once over the membraneous material around the support member. The membraneous material was folded along the core fiber. The core fiber was wound once over the two layers of the membraneous material around the support member. The top layer membraneous material was folded back. At this time, the space between the core fibers is desirably less than the diameter of the core fiber, while the space is not particularly critical as far as it is at least the thickness of the membraneous material. In this example, the space was 0.5 mm.

Next, the core fiber was wound once over the bottom layer membraneous material around the support member. The core fiber was then covered by the top layer membraneous material. This operation was repeated until the core fiber was wound around 20 times.

The whole body with the support member was immersed in distilled water and taken out immediately. Then the shape of the membraneous material was adjusted, and the whole body was air-dried. After the body was dried, a 2% aqueous gelatin solution was applied over the membraneous material, and gelatin was dried at 25°C for 3 hrs while gelatin was allowed to penetrate. Then, the body was heated at 120°C for 24 hrs for gelatin to cross-link. The core fiber outside the membraneous material on the support member was cut to remove the membraneous material and the core fibers were pulled out. Thus, a sheet with 20 hollow spaces was obtained.

By spirally winding the obtained sheet, a hollow bundle was obtained.

### Example 2

A hollow sheet with hollow spaces having an inside diameter of 1 mm was obtained by the same operation as in Example 1, except for using a bundle of five Teflon (trade-mark) fibers of 0.4-mm diameter as the core fiber. The use of a plurality of core fibers made the operation of pulling out core fiber easier. In addition, it became clear that the inside diameter of the hollow sheet could be established freely by modifying the number of the core fibers.

### Effects of the invention

The invention has made it possible to easily obtain a hollow fiber that may be useful as artificial neurilemma of a narrow diameter which was difficult to manufacture in the past. In addition, the inside diameter can now be regulated freely.

## Claims

1. A hollow fiber composed essentially of a membraneous material folded to form at least one open-ended cylindrical hollow space having an inside diameter of from 1 µm to 5 mm, wherein:
the membraneous material consists only of a cellular compact layer and basement membrane layer that are components of a membraneous connective tissue of a mammal;
the membraneous material is composed essentially of types I, III, IV, V and XVI collagen bundles; and
front and back surfaces of the membraneous material are asymmetrical with each other.

2. The hollow fiber according to claim 1, which is in a hollow sheet form having a cross-section composed of a straight line and a waved or corrugated line, both made of the membraneous material connected together at bottoms of waves of the waved or corrugated line, whereby a plurality of the open-ended cylindrical hollow spaces are formed by the straight line and the waved or corrugated line.

3. The hollow sheet according to claim 2, wherein the sheet is reinforced by gelatin, collagen or a medically acceptable adhesive on a waved or corrugated side of the sheet, whereby the cylindrical hollow spaces are also formed between the waved or corrugated membraneous material and the gelatin, collagen or medically acceptable adhesive.

4. The hollow sheet according to claim 3, wherein the membraneous connective tissue is of human.

5. The hollow sheet according to claim 4, wherein the membraneous connective tissue is human amnion.

6. The hollow sheet according to claim 3, wherein the cylindrical hollow spaces have an inner diameter of from 100 µm to 2 mm.

7. The hollow sheet according to claim 3, which has 2 to 50 of the cylindrical hollow spaces arranged in a row.

8. The hollow sheet according to claim 3, wherein the membraneous material is strengthened by a biodegradable and bioabsorbable fiber.

9. The hollow sheet according to claim 4, which is spirally wound.

10. A process for producing a hollow sheet, which comprises:
(A) loading a membraneous material on a support member;
(B) winding once a core fiber or a bundle of core fibers over the membraneous material and around the support member, wherein the core fiber or the bundle has a diameter of from 1 µm to 5 mm;
(C) folding back the membraneous material along the core fiber or the bundle;
(D) winding once the core fiber or the bundle over top and bottom layers of the folded back membraneous material and around the support member;
(E) lifting the top layer of the membraneous material, winding once the core fiber or the bundle over the bottom layer of the membraneous material and around the support member and placing back the top layer of the membraneous material onto the core fiber or the bundle;
(F) repeating the steps (D) and (E) a predetermined number of times, thereby obtaining an assembly of the membraneous material and the core fiber or bundle around the support member;
(G) applying an aqueous gelatin or collagen solution or a medically acceptable adhesive to the membraneous material and the core fiber or bundle;
(H) hardening the gelatin, collagen or medically acceptable adhesive;
(I) cutting the core fiber or bundle where only membraneous material is absent, whereby obtaining a composite of the membraneous material, the core fiber or bundle and the gelatin, collagen or medically acceptable adhesive, removed from the support member; and
(J) pulling out the core fiber or bundle from the composite, thereby obtaining the desired hollow sheet having a plurality of open-ended cylindrical hollow spaces having an inner diameter of 1 µm to 5 mm formed by the folded membraneous material and the gelatin, collagen or medically acceptable adhesive, the membraneous material consists only of a cellular compact layer and basement membrane layer that are components of a membraneous connective tissue of a mammal; and
the membraneous material is composed essentially of types I, III, IV, V and XVI collagen bundles; and
front and back surfaces of the membraneous material are asymmetrical with each other.
